# EUROPEAN PATENT APPLICATION

(11) **EP 2 474 324 A1**
(43) Date of publication of application: **11.07.2012**
(21) Application number: 10812081.7
(22) Date of filing: 31.08.2010
(51) Int. Cl.: A61K 47/32, A61K 9/32, A61K 9/36, A61K 47/38, C09D 101/00, C09D 101/26, C09D 129/04, C09D 151/08

(54) **COATING COMPOSITION**

(30) Priority: 31.08.2009 JP 2009200248
(71) Applicant: Nisshin Kasei Co., Ltd., Osaka 541-0045 (JP)
(72) Inventor: YOSHINO, Hiroyuki, Kobe-shi Hyogo 651-1513 (JP); MORIUCHI, Toshiaki, Yamatokoriyama-shi Nara 639-1058 (JP); KOJO, Akane, Kyoto-shi Kyoto 603-8212 (JP); HAYASHI, Yusuke, Kobe-shi Hyogo 651-1301 (JP)
(74) Representative: Müller-Boré & Partner Patentanwälte
(86) International application number: PCT/JP2010/064815
(87) International publication number: WO 2011/025035

(57) **Abstract**

A main object of the present invention is to provide a coating composition capable of maintaining an excellent moisture-proof property of coating compositions mainly comprising a polyvinyl alcohol polymer, as well as increasing the coating process speed and improving the productivity. The object is achieved by a coating composition comprising a polymer or copolymer (A) obtained by polymerizing or copolymerizing at least one polymerizable vinyl monomer in the presence of polyvinyl alcohol and/or a derivative thereof, and a cellulose-based polymer (B).

## Description

### Technical Field

The present invention relates to a composition comprising a polyvinyl alcohol polymer and a cellulose-based polymer, which is useful for coating of drugs and the like. More specifically, the present invention relates to a coating composition for drugs and the like, comprising a polymer or copolymer obtained by polymerizing or copolymerizing at least one polymerizable vinyl monomer in the presence of polyvinyl alcohol and/or a derivative thereof; and a cellulose-based polymer.

### Background Art

A polyvinyl alcohol copolymer obtained by copolymerizing at least one or more polymerizable vinyl monomers with polyvinyl alcohol is known to be useful as a major ingredient of hard capsules for poorly soluble medicinal substances (see Patent Literature 1). Further, a coating agent comprising the polyvinyl alcohol copolymer is known to exhibit excellent performance in terms of antioxidant effect, masking effect of unpleasant odors, and the like (see Patent Literature 2).

However, when the polyvinyl alcohol copolymer is the only main ingredient of a coating agent, the coating agent will be highly adhesive. This causes solid preparations such as tablets to adhere to the inside of a device and/or to form agglomerations during a coating process, or requires a long period of time for the coating process because the solution feed rate cannot be increased. As a result, it was not possible to achieve sufficient productivity.

### Citation List

### Patent Literature

[PTL 1] WO 2002/017848
[PTL 2] WO 2005/019286

### Summary of Invention

### Technical Problem

A main object of the present invention is to provide a coating composition capable of maintaining an excellent moisture-proof property of coating compositions mainly comprising a polyvinyl alcohol polymer or copolymer, as well as increasing the coating process speed and improving the productivity.

### Solution to Problem

The present inventors conducted intensive studies in light of the above problems; and, as a result, found that adding a cellulose-based polymer in addition to a polyvinyl alcohol copolymer to a coating composition results in the coating composition capable of reducing the time required for the coating process and having a moisture-proof property equivalent to that of coating agents consisting of a polyvinyl alcohol copolymer. The present invention has been completed based on such findings, and further studies.

Specifically, the present invention encompasses compositions and the like described in the following items.

### Item 1.

A coating composition comprising
(A) a polymer or copolymer obtained by polymerizing or copolymerizing at least one polymerizable vinyl monomer in the presence of polyvinyl alcohol and/or a derivative thereof; and
(B) a cellulose-based polymer.

### Item 2.

The coating composition according to Item 1, wherein the polymerizable vinyl monomer is selected from the group consisting of unsaturated carboxylic acids or salts thereof, and esters of unsaturated carboxylic acids.

### Item 3.

The coating composition according to Item 1, wherein the polymerizable vinyl monomer is a compound represented by Formula (1):

H₂C = C(R¹)-COOR² (1)

wherein R¹ represents hydrogen or methyl, and R² represents hydrogen or alkyl having 1 to 4 carbon atoms;
or a salt thereof.

### Item 4.

The coating composition according to Item 1, wherein the polymer or copolymer of (A) is a polymer or copolymer obtained by polymerizing or copolymerizing (I) at least one member selected from the group consisting of acrylic acid, methacrylic acid, their sodium salts, potassium salts, ammonium salts, and alkylamine salts; and (II) at least one polymerizable vinyl monomer selected from the group consisting of methyl methacrylate, methyl acrylate, ethyl methacrylate, ethyl acrylate, butyl methacrylate, butyl acrylate, isobutyl methacrylate, and isobutyl acrylate, in the presence of polyvinyl alcohol and/or a derivative thereof.

### Item 5.

The coating composition according to Item 4, wherein (II) is methyl methacrylate.

### Item 6.

The coating composition according to any of Items 1 to 5, wherein the cellulose-based polymer is at least one member selected from the group consisting of hydroxypropyl methyl cellulose, hydroxypropyl cellulose, methyl cellulose, and hydroxyethyl cellulose.

### Item 7.

The coating composition according to any of Items 1 to 6, wherein the ratio of (A) to (B) is about 1:0.02-2.5 by weight ratio.

### Item 8.

A coated preparation obtained by coating a solid preparation with the coating composition according to any of Items 1 to 7.

### Item 9-1.

A method for producing a coated preparation, comprising a step of applying the coating composition according to any of Items 1 to 7 to a solid preparation.

### Item 9-2.

A method for producing a coated preparation by applying the coating composition according to any of Items 1 to 7 to a solid preparation.

### Item 10-1.

A method for coating a solid preparation, comprising a step of applying the coating composition according to any of Items 1 to 7 to the solid preparation.

### Item 10-2.

A method for coating, comprising applying the coating composition according to any of Items 1 to 7 to a solid preparation.

### Item 11.

The coating composition according to any of Items 1 to 7 for use in coating a solid preparation.

### Item 12.

The coating composition according to any of Items 1 to 7 and 11 for use in spray coating.

### Advantageous Effects of Invention

The coating composition of the present invention is advantageous in that it has an excellent moisture-proof property, and increases the coating speed by reducing the adhesive property, thereby improving the productivity. When the coating composition of the present invention is used, it prevents the adhesion of preparations to a device and the agglomeration of preparations during coating, allowing a significant increase in the coating speed, compared to when a coating composition consisting of a polyvinyl alcohol polymer is used.

Further, the coating composition of the present invention has an excellent moisture-proof property equivalent to that of coating compositions consisting of a polyvinyl alcohol copolymer. The coating composition of the present invention also has a masking effect of unpleasant odors and bitter taste, and an effect of preventing oxygen permeation.

The present invention allows efficient production of coated preparations having excellent functions in a short period of time.

### Brief Description of Drawings

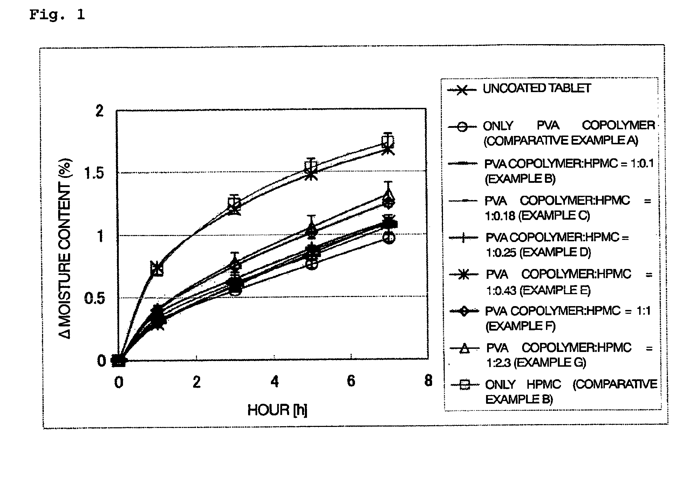

[Fig. 1] Preparations coated with each coating solution and uncoated tablets were dried in a dryer at 40°C for 2 days, and then stored under conditions of 25°C and 75% RH. The moisture absorption rate of each tablet was examined by measuring the increase in the moisture content in the tablet after the passage of each period of time. Fig. 1 shows the results of the examination based on the different amount of HPMC in the tablet. The ordinate in Fig. 1 represents the rate of increase in the moisture content when the moisture content immediately after coating is assumed to be 0%. The abscissa in Fig. 1 represents the passage of time since the storage.

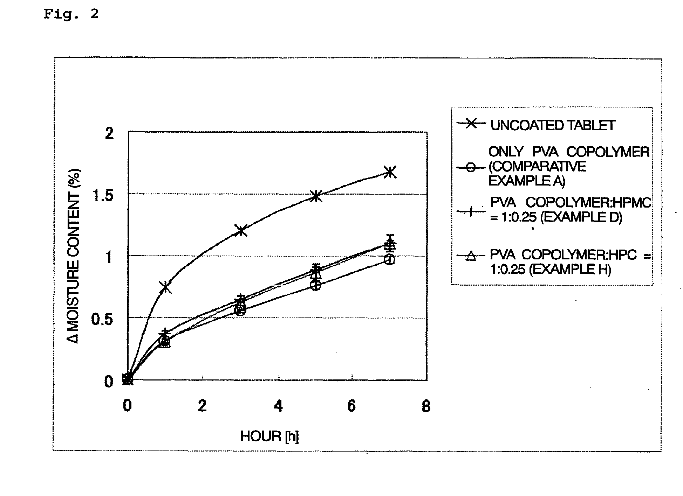

[Fig. 2] Preparations coated with each coating solution and uncoated tablets were dried in a dryer at 40°C for 2 days, and then stored under conditions of 25°C and 75% RH. The moisture absorption rate of each tablet was examined by measuring the increase in the moisture content in the tablet after the passage of each period of time. Fig. 2 shows the results of the examination based on the different component of the composition. The ordinate in Fig. 2 represents the rate of increase in the moisture content when the moisture content immediately after coating is assumed to be 0%. The abscissa in Fig. 2 represents the passage of time since the storage.

### Description of Embodiments

The present invention is described in more detail below.

### (1) Polymer or copolymer obtained by polymerizing or copolymerizing at least one polymerizable vinyl monomer in the presence of polyvinyl alcohol and/or a derivative thereof (hereinbelow also referred to as "polyvinyl alcohol polymer")

A polyvinyl alcohol polymer, which is one of the components of the coating composition of the present invention, can be produced by polymerizing or copolymerizing at least one polymerizable vinyl monomer by a known polymerization method-in the presence of polyvinyl alcohol or a derivative thereof. Examples of known polymerization methods include radical polymerization. Specific examples include solution polymerization, suspension polymerization, emulsion polymerization, and bulk polymerization.

Polymerization reaction is usually performed in the presence of a polymerization initiator, and, if necessary, a reducing agent, chain transfer agent, dispersant, or the like, in water, an organic solvent, or a mixture thereof.

Examples of reducing agents include sodium erythorbate, sodium metabisulfite, and ascorbic acid.

Examples of chain transfer agents include 2-mercaptoethanol, α-methylstyrene dimer, 2-ethylhexylthioglycolate, and laurylmercaptan. Examples of dispersants include surfactants such as sorbitan ester and lauryl alcohol.

Examples of organic solvents include lower alcohols such as methanol and ethanol, glycol ethers, and glycol esters. Removal of unreacted monomers can also be performed by a known method.

The polyvinyl alcohol used as a starting material of the polyvinyl alcohol polymer of the present invention is usually polyvinyl alcohol having an average degree of polymerization of about 100 to 2,000, preferably about 200 to 1,300, more preferably about 200 to 900, further more preferably about 200 to 600, and most preferably about 300 to 500.

Additionally, the degree of saponification of the polyvinyl alcohol is usually about 96 mol% or less, preferably about 78 to 96 mol%. The degree of saponification of the polyvinyl alcohol is defined as the ratio of hydroxyl groups introduced in a step of converting polyvinyl acetate into polyvinyl alcohol by replacing acetate groups in polyvinyl acetate with hydroxyl groups. Such a partially saponified polyvinyl alcohol can be produced by radically polymerizing vinyl acetate and suitably saponifying the resulting polyvinyl acetate. A desired polyvinyl alcohol can be produced by suitably controlling the degree of polymerization and the degree of saponification by known methods. It is possible to use commercially available products of these partially saponified polyvinyl alcohols. Examples of usable commercially available products include Gohsenol (registered trademark) EG05 (produced by The Nippon Synthetic Chemical Industry Co., Ltd.), EG25 (produced by The Nippon Synthetic Chemical Industry Co., Ltd.), PVA203 (produced by Kuraray Co., Ltd.), PVA204 (produced by Kuraray Co., Ltd.), PVA205 (produced by Kuraray Co., Ltd.), JP-04 (produced by Japan Vam & Poval Co., Ltd.), and JP-05 (produced by Japan Vam & Poval Co., Ltd.).

Further, examples of polyvinyl alcohol derivatives include various modified polyvinyl alcohols, for example, amine-modified polyvinyl alcohols, ethylene-modified polyvinyl alcohols, carboxylic acid-modified polyvinyl alcohols, diacetone-modified polyvinyl alcohols, and thiol-modified polyvinyl alcohols. Commercially available products of these modified polyvinyl alcohols may be used. Modified polyvinyl alcohols produced by a known method in the relevant field can also be used.

Polyvinyl alcohol or a derivative thereof used as a starting material of the polyvinyl alcohol polymer can be used singly or in a combination of two or more thereof. The ratio of these materials when used in combination can be suitably determined. For example, polyvinyl alcohol having an average degree of polymerization of 300 and polyvinyl alcohol having an average degree of polymerization of 1,500 can be suitably mixed and used as materials of the polyvinyl alcohol.

Examples of polymerizable vinyl monomers to be polymerized with polyvinyl alcohol include unsaturated carboxylic acids or salts thereof, esters of unsaturated carboxylic acids, unsaturated nitriles, unsaturated amides, aromatic vinyls, aliphatic vinyls, unsaturated bond-containing heterocycles, and salts thereof.

Examples of unsaturated carboxylic acids include acrylic acid, methacrylic acid, crotonic acid, fumaric acid, maleic acid, and itaconic acid.

Examples of salts of unsaturated carboxylic acids include alkali metal salts, ammonium salts, and alkylamine salts of the unsaturated carboxylic acid.

Examples of esters of unsaturated carboxylic acids include substituted or unsubstituted alkyl esters, cyclic alkyl esters, polyalkylene glycol esters of the unsaturated carboxylic acid.

Specific examples of esters of unsaturated carboxylic acids include acrylic acid esters such as methyl acrylate, ethyl acrylate, propyl acrylate, butyl acrylate, isobutyl acrylate, cyclohexyl acrylate, 2-ethylhexyl acrylate, hydroxyethyl acrylate, polyethyleneglycol acrylate (ester of polyethyleneglycol and acrylic acid), and polypropylene glycol acrylate (ester of polypropylene glycol and acrylic acid).

Specific examples of esters of unsaturated carboxylic acids also include methacrylic acid esters such as methyl methacrylate, ethyl methacrylate, propyl methacrylate, butyl methacrylate, isobutyl methacrylate, cyclohexyl methacrylate, 2-ethylhexyl methacrylate, hydroxyethyl methacrylate, and polyethyleneglycol methacrylate (ester of polyethyleneglycol and methacrylic acid).

Examples of unsaturated nitriles include acrylonitrile and methacrylonitrile.

Examples of unsaturated amides include acrylamide, dimethylacrylamide, and methacrylamide.

Examples of aromatic vinyls include styrene and α-methylstyrene.

Examples of aliphatic vinyls include vinyl acetate.

Examples of unsaturated bond-containing heterocycles include N-vinylpyrrolidone and acryloylmorpholine.

Of these examples, a preferable polymerizable vinyl monomer is a compound represented by Formula (1):

H₂C = C(R¹)-COOR² (1)

wherein R¹ represents hydrogen or methyl, and R² represents hydrogen or alkyl having 1 to 4 carbon atoms.
Specific examples include acrylic acid, methacrylic acid, methyl methacrylate, methyl acrylate, ethyl methacrylate, ethyl acrylate, propyl methacrylate, propyl acrylate, butyl methacrylate, butyl acrylate, isobutyl methacrylate, and isobutyl acrylate. In regard to acrylic acid and methacrylic acid, salts thereof can also be used. For example, their sodium salts, potassium salts, ammonium salts, or alkylamine salts may be used.

These polymerizable vinyl monomers may be used singly or in a combination of two or more thereof. For example, two vinyl monomers, such as an unsaturated carboxylic acid or a salt thereof and an unsaturated carboxylic acid ester, can be copolymerized with polyvinyl alcohol. In particular, in the present invention, it is preferable to copolymerize two or more polymerizable vinyl monomers with polyvinyl alcohol.

When two or more polymerizable vinyl monomers are used in combination, it is preferable to use
(I) at least one member selected from the group consisting of acrylic acid, methacrylic acid, their sodium salts, potassium salts, ammonium salts, and alkylamine salts; and
(II) at least one member selected from the group consisting of methyl methacrylate, methyl acrylate, ethyl methacrylate, ethyl acrylate, butyl methacrylate, butyl acrylate, isobutyl methacrylate, and isobutyl acrylate
   in combination.
   It is more preferable to use (I) acrylic acid and/or methacrylic acid and (II) methyl methacrylate in combination.

Further, when two or more polymerizable vinyl monomers are used, the ratio thereof is not particularly limited. For example, when (I) at least one member selected from the group consisting of acrylic acid, methacrylic acid, their sodium salts, potassium salts, ammonium salts, and alkylamine salts and (II) at least one member selected from the group consisting of methyl methacrylate, methyl acrylate, ethyl methacrylate, ethyl acrylate, butyl methacrylate, butyl acrylate, isobutyl methacrylate, and isobutyl acrylate are used in combination, the weight ratio of (I) to (II) is 0.5:9.5 to 5:5, preferably 1:9 to 4:6, and more preferably 1:9 to 3:7.

More specifically, for example, the weight percentage of (I) to the total weight of polymerizable vinyl monomers is preferably 5 to 50 wt%, and more preferably 10 to 40 wt%; and the weight percentage of (II) is preferably 50 to 95 wt%, and more preferably 60 to 90 wt%.

When acrylic acid and methyl methacrylate are used as polymerizable vinyl monomers, the weight ratio thereof is preferably about 3:7 to 0.5:9.5, and more preferably about 1.25:8.75.

Examples of preferable polyvinyl alcohol copolymers include a copolymer obtained by copolymerizing acrylic acid and methacrylic acid ester with polyvinyl alcohol. More specific examples include a copolymer obtained by copolymerizing acrylic acid and methyl methacrylate with polyvinyl alcohol.

The weight ratio of polyvinyl alcohol and polymerizable vinyl monomers (polyvinyl alcohol:polymerizable vinyl monomers) that are used is preferably about 6:4 to 9:1, and more preferably about 8:2.

Specifically, the polyvinyl alcohol polymer (A) preferably used in the coating composition of the present invention is a polyvinyl alcohol polymer obtained by copolymerizing methyl methacrylate and acrylic acid with polyvinyl alcohol having an average degree of polymerization of about 100 to 2000, wherein the weight ratio of polyvinyl alcohol, methyl methacrylate, and acrylic acid (polyvinyl alcohol:methyl methacrylate:acrylic acid) during copolymerization is about (60-90:7-38:0.5-12), particularly preferably about (80:17.5:2.5).

The weight ratio of polyvinyl alcohol and polymerizable vinyl monomers in the polyvinyl alcohol polymer is the same as that of polyvinyl alcohol and polymerizable vinyl monomers used for copolymerization. Consequently, the polymerization molar ratio of polyvinyl alcohol and polymerizable vinyl monomers in the polymer can be measured by NMR. Additionally, the molecular weights of polyvinyl alcohol and each polymerizable vinyl monomer are known. Therefore, even when the amount of each material used for copolymerization is unknown, it is possible to calculate the weight ratio of polyvinyl alcohol and polymerizable vinyl monomers based on the above information.

A known method can be used for copolymerization. The polyvinyl alcohol polymer of the present invention can be obtained, for example, by adding polyvinyl alcohol and/or its derivative to water followed by heating for dissolution, and adding at least one of the above-described polymerizable vinyl monomers and a polymerization initiator to the solution for copolymerization. For example, polyvinyl alcohol and/or its derivative is dispersed in ion-exchange water, and completely dissolved at 90 to 100°C. Subsequently, at least one of the above-described polymerizable vinyl monomers is added to the solution, nitrogen substitution is performed, and a polymerization initiator is added to perform reaction for about 2 to 5 hours. The weight ratio of polyvinyl alcohol and/or its derivative and polymerizable vinyl monomer in the polyvinyl alcohol polymer (A) described above is determined by the weight ratio of polyvinyl alcohol and/or its derivative and polymerizable vinyl monomer, which are added to water. Accordingly, the weight ratio of the materials added to the water is preferably the same as "the weight ratio of polyvinyl alcohol and/or its derivative and polymerizable vinyl monomer in polyvinyl alcohol polymer (A)" described above.

Any polymerization initiator used in the relevant field can be used as the polymerization initiator. Examples include inorganic peroxides such as potassium persulfate, ammonium persulfate, and hydrogen peroxide; organic peroxides such as peracetic acid and tert-butyl hydroxy peroxide; and azo compounds.

A commercially available product can also be used as the polyvinyl alcohol polymer of the present invention. For example, POVACOAT (registered trademark) Type F (produced by Daido Chemical Corporation) or the like can be used.

### (2) Cellulose Polymer

A polymer obtained by esterification or etherification of hydroxyl groups in cellulose are preferably used as a cellulose-based polymer.

Specific examples include hydroxypropyl methyl cellulose (also referred to as hypromellose, according to the Japanese Pharmacopoeia; hereinafter also referred to as "HPMC"), hydroxypropyl cellulose (hereinafter also referred to as "HPC"), methyl cellulose (hereinafter also referred to as "MC"), and hydroxyethyl cellulose (hereinafter also referred to as "HEC"). These may be used singly or in a combination of two or more thereof.

HPMC and HPC are particularly preferable in terms of the effect of reducing the adhesive property.

The weight-average molecular weight of cellulose-based polymer is not particularly limited insofar as the effects of the present invention are achieved. It is usually about 10,000 to 200,000 g/mol, and preferably about 20,000 to 50,000 g/mol. When it is 10,000 g/mol or more, it is preferable in terms of the adhesion reduction effect and the improvement of drying efficiency. Further, when it is 200,000 g/mol or less, operational problems due to an increase in the viscosity of the coating solution are less likely to occur. The above weight-average molecular weight is measured by GPC.

Further, the degree of esterification or etherification of cellulose-based polymer is not particularly limited insofar as the effects of the present invention are not adversely affected. Particularly when HPMC is used, the percentages of substituents introduced into the cellulose are preferably 19 to 33% of methoxy and 4 to 12% of hydroxypropoxy, and more preferably 28 to 30% of methoxy and 7 to 12% of hydroxypropoxy, when the percentage of total hydroxyl groups in the cellulose is assumed to be 100%.

These cellulose-based polymers are known, or can be easily produced by a known method. Commercially available cellulose-based polymers can also be purchased and used. For example, commercially available cellulose-based polymers can be purchased from Shin-Etsu Chemical Co., Ltd., Nippon Soda Co., Ltd., and the like.

Because a cellulose-based polymer is contained, the coating composition of the present invention can maintain the moisture-proof property possessed by the above coating compositions consisting of polyvinyl alcohol polymer, as well as achieving the effects of increasing the coating process speed and improving the productivity of coated preparations.

### (3) Coating Composition

The coating composition of the present invention is characterized by comprising the polymer or copolymer (A) obtained by polymerizing or copolymerizing at least one polymerizable vinyl monomer in the presence of polyvinyl alcohol and/or a derivative thereof (i.e., polyvinyl alcohol polymer); and the cellulose-based polymer (B).

The ratio of (A) and (B) is not particularly limited insofar as the effects of the present invention are achieved. The weight ratio of (A):(B) is usually 1:0.02-2.5, preferably 1:0.05-2.3, and more preferably about 1:0.1-0.7. When the ratio of (B) to (A) is 0.02 or more, the adhesive property is reduced, and it is thus preferable in terms of improving the productivity. Further, when the ratio is 2.5 or less, the moisture-proof function is less likely to be reduced. In particular, when the ratio is 0.7 or less, the moisture-proof function is further less likely to be reduced.

Additionally, the amount of polyvinyl alcohol polymer (A) in the coating composition of the present invention is also not particularly limited insofar as the effects of the present invention are achieved. The amount thereof relative to the total amount of the composition is usually 30 to 98 wt% and preferably about 60 to 90 wt%, in terms of solids content. When it is about 98 wt% or less, it is preferable in terms of improving the productivity. Additionally, when it is about 30 wt% or more, the moisture-proof function is less likely to be reduced.

Further, the amount of cellulose-based polymer (B) in the coating composition of the present invention is also not particularly limited insofar as the effects of the present invention are achieved. The amount thereof relative to the total amount of the composition is usually 2 to 70 wt% and preferably about 10 to 40 wt%, in terms of solids content. When it is about 2 wt% or more, it is preferable in terms of improving the productivity. Further, when it is about 70 wt% or less, the moisture-proof function is less likely to be reduced.

The form of the composition of the present invention is also not particularly limited. For example, it may be a composition comprising (A) and (B), or a composition obtained by adding (A) and (B) to a solvent. In other words, it may be a composition in which (A), (B), and a solvent are mixed, or the like. Examples of solvents include water, hydrophilic organic solvents, or mixtures thereof. Examples of hydrophilic organic solvents include alcohols such as ethanol; and polyols such as glycerin and polyethyleneglycol. The mixture ratio of water and hydrophilic organic solvent is not particularly limited, and can be suitably determined. For example, the weight ratio of water to hydrophilic organic solvent may be about 1:0.1-10. Generally, in the actual application of the coating composition to drugs, animal drugs, pesticides, fertilizers, food products, and the like, the coating composition is preferably used for coating by spraying, atomizing, and like other means in the form of aqueous solution, aqueous dispersion, organic solvent solution, or organic solvent dispersion.

Although not particularly limited, when the total weight of (A) and (B) is assumed to be 1, the weight ratio of the solvent to (A) and (B) is preferably about 5-50:1, and more preferably about 10-40:1.

Further, the coating composition of the present invention can contain components other than (A) and (B) within the range in which the effects of the present invention are achieved.

Examples of other components include agglomeration inhibitors, dyes, plasticizers, lubricants, flavoring substances, and aromas. Examples include titanium dioxide, talc, magnesium stearate, various edible dyes, various sweeteners having high degree of sweetness such as aspartame, sucralose, acesulfame-K, and stevia, various aromas such as mint flavor, various fruit flavors, chocolate flavor, coffee flavor, tea flavor, and vanilla flavor, various amino acids, various organic acids such as citric acid, malic acid, succinic acid, lactic acid, glyconic acid, tartaric acid, acetic acid, acetic anhydride, adipic acid, maleic acid, fumaric acid, ascorbic acid, alginic acid, gluconic acid, and nicotinic acid, and salts thereof. These may be used singly or in a combination of two or more thereof.

Coating methods of the coating composition of the present invention include application. Specifically, the coating composition can be applied by, for example, spraying, dipping, and the like. In particular, application by spraying is preferable. Spray application can be performed by a method using a known apparatus. For example, any standard method using a pan coating apparatus, drum-type coating apparatus, or fluid-coating apparatus may be used. These are preferably vent-type coating apparatuses. Generally widely used apparatuses such as fluidized-bed coating apparatuses, rolling fluidized-bed coating apparatuses, tablet coating apparatuses, and automatic coating apparatuses can be suitably used according to the dosage form to be coated. Specific examples of fluidized-bed coating apparatuses include a spouted-bed coating apparatus.

Products to which the coating composition of the present invention is applicable are also not particularly limited. For example, the coating composition is applicable to solid preparations, health food products, food products, and the like. In particular, the present invention can be suitably used for coating of solid preparations.

Examples of solid preparations include tablets, granules, powders, pills, and capsules.

For example, the coating composition of the present invention can be used for film coating of tablets.

The coating composition of the present invention can reduce the adhesive property of the coating, increase the coating speed, and improve the productivity of coated preparations. Further, the coating composition of the present invention also has properties possessed by coating compositions consisting of a polyvinyl alcohol copolymer, and is particularly excellent in moisture-proof property.

Still further, the coating composition of the present invention has a masking effect of unpleasant odors in drugs, animal drugs, pesticides, fertilizers, food products, and the like. Examples of such unpleasant odors include unpleasant or pungent odors peculiar to products derived from drugs (such as L-cysteine, thiamin hydrochloride, methionine, digestive enzyme preparation, and various crude drugs) or pesticides, and unpleasant odors derived from various food products (such as fishy odor, retort odor, and animal meat odor). The present invention is effective in suppressing such unpleasant odors. The present invention also has a masking effect of bitter taste of drugs, food products, and the like. Examples of drugs having a bitter taste are not particularly limited, but include acetaminophen, pyridoxine hydrochloride, anhydrous caffeine, chlorpromazine, erythromycin, phenobarbital, and promethazine hydrochloride. Further, the application of the coating composition of the present invention to unstable drugs that may interact with other drugs can prevent such interactions. Examples of such unstable drugs that may interact with other drugs include isopropylantipyrine and acetaminophen which give a depression in melting point when mixed together; and phenylpropanolamine and chlorpheniramine maleate which give a color change when mixed together. Still further, the coating composition of the present invention also has an effect of preventing oxygen permeation, and is therefore useful for coating of drugs (such as ascorbic acid, vitamin A; and vitamin E), animal drugs, pesticides, fertilizers, and food products, which are susceptible to oxidative degradation.

### (4) Coated Preparation

The coated preparation of the present invention is a preparation, such as a solid preparation, whose surface is coated with the coating composition of the present invention.

Examples of solid preparations include drugs, animal drugs, pesticides, fertilizers, and food products. Preferable examples include solid preparations, such as tablets, granules, powders, pills, and capsules, described in the General Rules for Preparations of the Japanese Pharmacopoeia, 15th Edition.

Solid preparations can be produced by a standard pharmaceutical method. For example, tablets can be produced by a direct powder compression method, dry granule compression method, semi-dry granule compression method, or wet granule compression method.

Methods for coating solid preparations with the coating composition of the present invention are also not particularly limited. Solid preparations can be coated by a standard pharmaceutical method. Examples include a pan coating method, a fluidized-bed coating method, and a rolling fluidized-bed coating method.

Further, the coating amount of the coating composition of the present invention can be suitably determined according to the preparation size and the like, and is usually about 1 to 100%, particularly about 2 to 50%, in terms of weight percent of the coating composition relative to the amount of preparation before coating.

Additionally, drugs contained in the preparation of the present invention are also not particularly limited insofar as the effects of the present invention are achieved. Examples include one or two or more components selected from revitalizers, antipyretic analgesic antiphlogistics, psychotropics, anxiolytics, antidepressants, hypnosedatives, anticonvulsants, central nervous system drugs, cerebral metabolism improving agents, cerebral circulation improvers, anticonvulsants, sympathetic agents, gastrointestinal drugs, acid suppressants, anti-ulcerogenic drugs, cough medicines, antiemetics, respiratory stimulants, bronchodilators, allergy drugs , dental and oral agents, antihistamines , cardiotonics, drugs for arrhythmia, diuretics, blood pressure-lowering drugs, vasoconstrictors, coronary dilators, peripheral vasodilators, drugs for hyperlipidemia, cholagogues, antibiotics, chemotherapeutic agents, drugs for diabetes mellitus, drugs for osteoporosis, anti-rheumatic drugs, skeletal muscle relaxants, anticonvulsants, hormone drugs, alkaloidal narcotics, sulfa drugs, antipodagrics, anti-blood clotting agents, and anti-malignant tumor agents.

Furthermore, in addition to the above drugs, known additives such as agglomeration inhibitors, dyes, plasticizers, lubricants, flavoring substances, and aromas can also be added to the preparation of the present invention.

While the coated preparation of the present invention achieves improved productivity, the coated preparation also has properties possessed by coating solutions consisting of a polyvinyl alcohol copolymer, and is particularly excellent in moisture-proof property. The coated preparation of the present invention can also provide the above-described effects.

### Examples

. The present invention is described in further detail below with reference to Examples and Comparative Examples. However, the present invention is not limited to these Examples.

### 1. Coating Composition

### Example A

In a separable flask equipped with a reflux condenser, dropping funnel, thermometer, nitrogen inlet tube, and stirrer, 175.8 g of polyvinyl alcohol (EG05; average degree of polymerization: 500, degree of saponification: 88%; produced by Nippon Synthetic Chemical Industry Co., Ltd.) and 582.3 g of ion-exchanged water were placed, dispersed at room temperature, and then completely dissolved therein at 95°C. Subsequently, 5.4 g of acrylic acid and 37.3 g of methyl methacrylate were added to the solution, followed by nitrogen substitution. After increasing the temperature to 50°C, 8.5 g of tertiary butyl hydro peroxide and 8.5 g of sodium erythorbate were added to the solution, and the reaction was terminated after 4 hours. The reaction product was dried and pulverized to obtain a polyvinyl alcohol copolymer (powder). Hereinafter, the polyvinyl alcohol copolymer is also described as a PVA copolymer. In the PVA copolymer, the weight ratio of polyvinyl alcohol:methyl methacrylate:acrylic acid is about 80:17.5:2.5. 4.75 g of the PVA copolymer and 0.25 g of hydroxypropyl methyl cellulose (TC-5R (registered trademark); produced by Shin-Etsu Chemical Co., Ltd.; weight-average molecular weight: 35,600 g/mol) were dissolved in 62.5 g of purified water to produce a coating solution. The percentages of substituents introduced into the hydroxypropyl cellulose used are about 29% of methoxy and about 10% of hydroxypropoxy, when the percentage of total hydroxyl groups in the cellulose is assumed to be 100%.

### Example B

A coating solution was produced in the same manner as in Example A, except that the amount of the PVA copolymer was 4.5 g instead of 4.75 g, and the amount of HPMC was 0.5 g instead of 0.25 g.

### Example C

A coating solution was produced in the same manner as in Example A, except that the amount of the PVA copolymer was 4.25 g instead of 4.75 g, and the amount of HPMC was 0.75 g instead of 0.25 g.

### Example D

A coating solution was produced in the same manner as in Example A, except that the amount of the PVA copolymer was 4.0 g instead of 4.75 g, and the amount of HPMC was 1.0 g instead of 0.25 g.

### Example E

A coating solution was produced in the same manner as in Example A, except that the amount of the PVA copolymer was 3.5 g instead of 4.75 g, and the amount of HPMC was 1.5 g instead of 0.25 g.

### Example F

A coating solution was produced in the same manner as in Example A, except that the amount of the PVA copolymer was 2.5 g instead of 4.75 g, and the amount of HPMC was 2.5 g instead of 0.25 g.

### Example G

A coating solution was produced in the same manner as in Example A, except that the amount of the PVA copolymer was 1.5 g instead of 4.75 g, and the amount of HPMC was 3.5 g instead of 0.25 g.

### Example H

4.0 g of PVA copolymer and 1.0 g of hydroxypropyl cellulose (NISSO HPC-L (registered trademark); produced by Nippon Soda Co., Ltd.) were dissolved in 62.5 g of purified water to produce a coating solution.

### Comparative Example A

5.0 g of PVA copolymer was dissolved in 62.5 g of purified water to produce a coating solution.

### Comparative Example B

5.0 g of HPMC (TC-5R (registered trademark); produced by Shin-Etsu Chemical Co., Ltd.; weight-average molecular weight: 35,600 g/mol) was dissolved in 62.5 g of purified water to produce a coating solution.

### 2. Coating Method

Using a spouted-bed coating apparatus equipped with a draft tube (Grow Max 140; produced by Fuji Paudal Co., Ltd.), coating was performed on 50 g of uncoated tablets having a tablet diameter Φ of .8.1 mm and a weight of 195 mg.

The coating conditions are as follows.

Supply air temperature: 60°C; supply air volume: 0.96 m³/minute; exhaust gas temperature: 45°C; spray air pressure: 0.08 MPa; and height of draft tube: 140 mm.

### 3. Evaluation of Coating Speed

Using the coating solutions of Examples A to G and Comparative Example A, the speed of each coating solution to coat the above tablets was compared and examined. Table 1 shows the results. The coating was regarded as finished when the coating solution was exhausted. '

The results were visually determined in accordance with the following evaluation criteria. The term "agglomeration" used herein refers to a state in which the tablets are adhered to each other, and a cluster comprising two or more tablets is formed.

I: There is no adhesion of tablets. Coating is smoothly performed.

II: Tablets sometimes adhere to the inner wall of the draft tube. Agglomeration occurs slightly, or the agglomeration rate is 10% or lower. The term "agglomeration rate" used herein refers to the percentage of the weight of lumps comprising two or more tablets, relative to the weight of all tablets.

III: Tablets continuously adhere to the inner wall of the draft tube. Agglomeration occurs, and the tablets stop moving. As a result, coating cannot be performed.

Further, in Table 1, the ratio of PVA copolymer:HPMC or HPC shows an approximate ratio of HPMC or HPC to the PVA copolymer in terms of weight.

**Table 1**

| Coating Solution Speed [mL/min] | Comparative Example A only PVA copolymer | Example A PVA copolymer: HPMC =1:005 | Example B PVA copolymer: HPMC =1:01 | Example C PVA copolymer: HPMC =1:018 | Example D PVA copolymer: HPMC =1:25 | Example E PVA copolymer: HPMC =1:043 | Example F PVA copolymer: HPMC =1:1 | Example G PVA copolymer: HPMC =1:23 | Example H PVA copolymer: HPC =1:0.25 |
|---|---|---|---|---|---|---|---|---|---|
| 0.7 | I | I | I | I | I | I | I | I | I |
| 1.0 | II | I | I | I | I | I | I | I | I |
| 1.2 | II | II | II | I | I | I | I | I | I |
| 1.3 | III | III | II | I | I | I | I | I | I |
| 1.4 | III | III | II | I | I | I | I | I | II |
| 1.7 | III | III | III | II | I | I | I | I | III |
| 1.9 | III | III | III | III | II | I | I | I | III |
| 2.3 | III | III | III | III | III | II | I | I | III |
| 2.4 | III | III | III | III | III | III | II | I | III |
| 2.7 | III | III | III | III | III | III | II | I | III |
| 3.4 | III | III | III | III | III | III | III | II | III |

As shown in Comparative Example A in Table 1, when the coating composition that does not comprise a cellulose-based polymer is used, agglomeration of tablets and adhesion of tablets to the inside of the device occur due to an increase in the coating speed; therefore, it has been difficult to increase the coating speed.

In contrast, when the coating solution in which a PVA copolymer and a cellulose-based polymer are used in combination is used, agglomeration of tablets during coating is prevented, allowing the coating speed to be increased. In particular, Example D shows that the coating speed can be two or more times faster than that of Comparative Example A, and Example F shows that the coating speed can be three or more times faster than that of Comparative Example A.

In Comparative Example A, coating until film-coated tablets with good appearance were obtained took 100 minutes. Specifically, in order to perform coating using Comparative Example A, without causing agglomeration of tablets or adhesion of tablets to the inside of the device (in other words, to perform coating to obtain film-coated tablets with good appearance), the speed of the coating solution to be sprayed must be reduced. Consequently, it took about 100 minutes until coating was finished.

In contrast, in the Examples, coating took about 65 minutes in Example B, about 45 minutes in Example D, and about 30 minutes Example F until film-coated tablets with good appearance were obtained. Specifically, when the coating solutions of Examples were used, it was possible to perform coating without causing agglomeration of tablets or adhesion of tablets to the inside of the device, even when the speed of the coating solution to be sprayed was increased; and it was possible to finish coating more quickly than when the coating solution of Comparative Example A was used. As is clear from the description of the production method in each example above, the amount of solution used for coating in each example is substantially the same.

As described above, it was confirmed that the productivity of coated tablets can be significantly improved by the use of the coating composition of the present invention, in which a PVA copolymer and a cellulose-based polymer are used in combination.

### 4. Evaluation of the Moisture-Proof Function of Coated Tablets

The coated tablets that were coated with the coating solutions of Examples A to H and Comparative Examples A and B described above were dried in a dryer at 40°C for 2 days, and then stored under conditions of 25°C and 75% RH (relative humidity). An increase in the moisture content in the tablets after the passage of each period of time was calculated from the change in the weight.

Fig. 1 shows the results obtained by analyzing the difference in the moisture absorption rate of the coated tablets depending on the amount of HPMC contained in the coated tablets. Further, Fig. 2 shows the results obtained by analyzing the difference in the moisture absorption rate of the coated tablets depending on the components contained in the coated tablets. The "Δ moisture content (%)" in Figs. 1 and 2 represents the rate of increase in the moisture content. Specifically, it represents the rate of increase in the weight, when the weight of each coated tablet immediately after drying is assumed to be 100%.

As shown in Fig. 1, it was found that the moisture absorption rate of the tablets coated with the coating solutions in the Examples of the present invention was as low as that of the tablets coated with the coating solution in Comparative Example A, in which a PVA copolymer was used singly. Further, as Fig. 2 shows, it was found that the moisture absorption rate was also equally low when HPC was used instead of HPMC.

Based on the above, it was confirmed that the excellent moisture-proof function possessed by the coating composition comprising a PVA copolymer was maintained, even when a cellulose-based polymer was added to the coating composition.

## Claims

1. A coating composition comprising a polymer or copolymer (A) obtained by polymerizing or copolymerizing at least one polymerizable vinyl monomer in the presence of polyvinyl alcohol and/or a derivative thereof; and a cellulose-based polymer (B).

2. The coating composition according to claim 1, wherein the polymerizable vinyl monomer is selected from the group consisting of unsaturated carboxylic acids or salts thereof, and esters of unsaturated carboxylic acids.

3. The coating composition according to claim 1, wherein the polymerizable vinyl monomer is a compound represented by Formula (1) below:
H₂C = C(R¹)-COOR² (1)
wherein R¹ represents hydrogen or methyl, and R² represents hydrogen or C^{l-4} alkyl;
or a salt thereof.

4. The coating composition according to claim 1, wherein the polymer or copolymer (A) is a polymer or copolymer obtained by polymerizing or copolymerizing (I) at least one member selected from the group consisting of acrylic acid, methacrylic acid, their sodium salts, potassium salts, ammonium salts, and alkylamine salts; and (II) at least one polymerizable vinyl monomer selected from the group consisting of methyl methacrylate, methyl acrylate, ethyl methacrylate, ethyl acrylate, propyl methacrylate, propyl acrylate, butyl methacrylate, butyl acrylate, isobutyl methacrylate, and isobutyl acrylate, in the presence of polyvinyl alcohol and/or a derivative thereof.

5. The coating composition according to claim 4, wherein (II) is methyl methacrylate.

6. The coating composition according to any of claims 1 to 5, wherein the cellulose-based polymer is at least one member selected from the group consisting of hydroxypropyl methyl cellulose, hydroxypropyl cellulose, methyl cellulose, and hydroxyethyl cellulose.

7. The coating composition according to any of claims 1 to 6, wherein the ratio of (A) to (B) is about 1:0.02-2.5 by weight ratio.

8. A coated preparation obtained by coating a solid preparation with the coating composition according to any of claims 1 to 7.

9. A method for producing a coated preparation, comprising a step of applying the coating composition according to any of Items 1 to 7 to a solid preparation.

10. A method for coating a solid preparation, comprising a step of applying the coating composition according to any of Items 1 to 7 to the solid preparation.
